(19) **Europäisches Patentamt**

**European Patent Office**

**Office européen des brevets**

(11) **EP 1 171 034 B1**

(12) **EUROPÄISCHE PATENTSCHRIFT**

(45) Veröffentlichungstag und Bekanntmachung des Hinweises auf die Patenterteilung:
**24.08.2005 Patentblatt 2005/34**

(51) Int Cl.⁷: **A61B 5/11**

(21) Anmeldenummer: **00918881.4**

(86) Internationale Anmeldenummer:
**PCT/EP2000/003378**

(22) Anmeldetag: **14.04.2000**

(87) Internationale Veröffentlichungsnummer:
**WO 2000/064347 (02.11.2000 Gazette 2000/44)**

(54) **VORRICHTUNG ZUR ERMITTLUNG DES HALSBEWEGUNGSMUSTERS**

DEVICE FOR ESTABLISHING A PATTERN OF MOVEMENT OF THE NECK

DISPOSITIF POUR LA DETERMINATION D'UN MODELE POUR LES MOUVEMENTS DU COU

(84) Benannte Vertragsstaaten:
**AT BE CH CY DE DK ES FI FR GB GR IE IT LI LU MC NL PT SE**

(30) Priorität: **21.04.1999 DE 19918008**

(43) Veröffentlichungstag der Anmeldung:
**16.01.2002 Patentblatt 2002/03**

(73) Patentinhaber: **Claussen, Claus-Frenz**
**97688 Bad Kissingen (DE)**

(72) Erfinder: **Claussen, Claus-Frenz**
**97688 Bad Kissingen (DE)**

(74) Vertreter: **Tergau & Pohl Patentanwälte**
**Mögeldorfer Hauptstrasse 51**
**90482 Nürnberg (DE)**

(56) Entgegenhaltungen:
**DE-A- 3 829 885**

**Beschreibung**

Die Erfindung bezieht sich auf eine Vorrichtung zur Ermittlung des Halsbewegungsmusters eines Probanden.

Seit geraumer Zeit besteht der Wunsch, eine geeignete Untersuchungsmethode zu finden, um Schädigungen der Halsbeweglichkeit infolge eines Kopf-Hals-Schleudertraumas, dem sogenannten HWS-Schleudertrauma, zuverlässig messen zu können. Vornehmliches Ziel der im körperlichen und funktionellen Bereich beschwerde- und einzelfallorientiert anzuwendenden Untersuchungsmethoden oder Verfahren ist die Aufklärung von unfallbedingten Verletzungen im Bereich der Halswirbelsäule.

Bei dem zu den häufigsten traumatischen Verletzungen des Halses zählenden HWS-Schleudertrauma oder Whiplash (Peitschenschlag)-Syndrom handelt es sich um eine krankheitsbildende Schädigung, die insbesondere infolge von Automobilunfällen mit und ohne Fahrzeuginnenkontakt auftritt. Das HWS-Schleudertrauma bezeichnet dabei ein physikalisches Beschleunigungs- oder Entschleunigungstrauma mit einem Energietransfer auf die zu einem nach Art eines Pendelstabes verbundene Kopf-Hals-Struktur mit einem oben aufgesetzten Dreh-GleitGelenk, nämlich dem Kopfsockelgelenk. Die Schleuderbewegungen können ausgelöst werden durch lineare Heckauffahr- bzw. Frontalaufprallunfälle oder durch lineare rechtsseitige oder linksseitige Aufprallunfälle. Zusätzlich sind auch vergleichsweise komplizierte Kollisionsmechanismen bekannt, bei denen die Fahrzeuge diagonal getroffen werden.

Bei den Diagonalkollisionen ergibt sich in der Regel eine Rotation des Fahrzeugs mit angulären Drehbeschleunigungen, die sich auch auf die Halswirbelsäulenstruktur, deren Gelenke, Bänder, Muskeln, Sehnen und übrigen Bewegungsverbindungen des oder jedes Fahrzeuginsassen fortsetzt. Das HWS-Schleudertrauma ist daher durch eine vielfältige und im Einzelfall durch das einwirkende Trauma häufig nur schwer vorhersehbare Symptomatik gekennzeichnet. In der medizinischen Literatur werden diesbezüglich verschiedene Begriffe zur Beschreibung dieses Schadens verwendet, wie z. B. cervico-encephales, -brachiales, -medulläres oder cervikales Syndrom, chronisches posttraumatisches Kopf-Hals-Leiden, HWS-Distorsion oder Whiplash-Syndrom.

Da der Halsbereich nicht nur von dem Rückenmark als wichtigen zentralnervösen Steuerorgan, sondern auch von wichtigen Blutgefäßen, Nervenbahnen, Muskeln, Sehnen, der Speise- und Luftröhre durchzogen wird, können Verletzungen dieses Bereiches zu besonderes schweren und durch eine Vielzahl von Symptomen bestimmten Krankheitsbildern führen. Die Multiplizität dieser vielen Symptome und Syndrome hat dazu geführt, dass es bisher keinen in einfacher Weise aufzufindenden und gleichzeitig zuverlässigen diagnostischen Befund gibt, der das HWS-Schleudertrauma an sich beweisend aufzeigen kann.

Ein erster Nachweis ist durch eine radiologische Bestimmung der Bänderverletzungen innerhalb des Kopfsockelgelenkes, d. h. der Verbindungen der Schädelbasis mit dem ersten und zweiten Halswirbel, gelungen. Dabei handelt es sich um ein kernspintomographisches Meßverfahren, welches letztendlich jedoch nur größere morphologische Verletzungen, wie Bänderzerreißungen, Bändereinrisse, Bänderverschwielungen und Exzentrizitäten bzw. Frakturen des Dens axis, nachweisen können.

Die Betroffenen (Patienten) selbst bemerken die erheblichen Veränderungen im Kopfsockelgelenk nach dem Zusammenprall mit einem Fahrzeug, indem sie einen steifen Nacken bekommen, an Nackenschmerzen leiden und in ihrer Kopf-Halsbeweglichkeit erheblich eingeschränkt sind.

Bei einem aus der WO 91/15148 (PCT/US 91/01796) bekannten nicht-invasiven Verfahren zur Bestimmung der Bewegung der Halswirbelsäule wird ein sitzend positionierter Proband angewiesen, Sehstimuli und systematisch einem leuchtenden Markierungsmuster an einer Wand nachzublicken, während seine Kopfbewegungen mittels Videokameras dreidimensional aufgezeichnet werden. Dazu ist am Kopf des Probanden eine aufzeichenbare Markierungseinrichtung angebracht. Werden Abweichungen von der geraden Folge der Leuchtsimulatoren ermittelt, so werden diese Abweichungen als Fehlsteuerungen der Halsbewegungen gedeutet.

Obwohl die Untersuchung am sitzenden Patienten durchgeführt wird, ist bei diesem bekannten Verfahren einerseits nicht sichergestellt, dass sich der Patient oder Proband bei den Kopfbewegungen derart ruhig verhält, dass keine zusätzlichen Kopf- und Rumpfbewegungen auftreten, die unter natürlichen Bedingungen die zielgerichtete Kopfbewegung unterstützen. Andererseits führt die Sitzposition in nachteiliger Weise zu einer Einschränkung der Bewegung des Probanden. Da somit die Kopfbewegung unterstützende Rumpfbewegungen nicht berücksichtigt werden und lediglich aus der erfassten Kopfbewegung auf die Bewegung oder Beweglichkeit der Halswirbelsäule geschlossen wird, ohne darüber hinaus zwischen unterer (Pendelstab) und oberer (Kopfsockelgelenk) HWS zu unterscheiden, ist das bekannte Verfahren insbesondere im Hinblick auf eine zuverlässige Aussage über die Art und den Grad sowie bezüglich der Lokalisierung einer Schädigung der Halswirbelsäule nicht ausreichend exakt. Daher kann mit diesem bekannten Verfahren die Kausalität einer Verletzung der Halswirbelsäule infolge eines durch einen Verkehrsunfall bedingten HWS-Schleudertraumas zumindest nicht in der gewünschten Weise fundiert nachgewiesen werden.

Bei einer aus der in der Schriftreihe des Hauptverbandes der gewerblichen Berufsgenossenschaften e.V. erschienenen Druckschrift "Forschungsbericht Cranio-Corpo-Grafie (CCG)", ISBN 3-88383-126-3 (Juni 1986), bekannten Einrichtung wird die Kopf- und Rumpfbewegung eines Probanden mittels Markierern in Form von Glühlampen sichtbar gemacht, von welchen je einer an beiden Schultern des Probanden und über dessen Stirn sowie dessen Hinterhaupt angebracht ist. Die Bewegung jedes Markierers in der horizontalen Ebene wird von einer über dem Probanden angeordneten Kamera in Dauerbelichtung als Leuchtspur in einem sogenannten Cranio-Corpo-Gramm fotografisch festgehalten. Die Leuchtspuren werden nach Durchführung des Experimentes auf der Fotografie ausgewertet. Die entsprechende Auswertung der Aufnahmen, die entweder durch Vermessen der Geometrie der Leuchtspuren oder durch assoziative Verbindung des komplexen Bewegungsmusters mit als "Grafo-Element" bezeichneten Vergleichsmustern erfolgt, ist jedoch mit einem erheblichen Zeitaufwand verbunden. Ein weiterer Nachteil besteht darin, dass bei der fotografischen Aufnahme der Markiererbewegungen ein Teil der im Experiment erzeugten Informationen verloren ist, zumal auf der Fotografie lediglich die horizontalen Komponenten der Markiererbewegungen zu erkennen sind. Eine Aussage über vertikale Bewegungen und über die absolute Höhe eines Markierers im Raum kann somit nicht getroffen werden. Darüber hinaus kommt es häufig zu Abdeckungen durch Überschneidungen der Leuchtspuren, da die Leuchtspuren aller Markierer auf einer einzelnen Fotografie enthalten sind. Eine Charakterisierung einzelner Leuchtspuren ist somit erschwert oder sogar unmöglich. Information geht auch in dem direkt unterhalb der Kammer gelegenen toten Winkel verloren, in welchem die Kammer in den zwischen dem Spiegel und einem Markierer verlaufenden Strahlengang hineinsteht.

Aus der DE 38 29 885 C2 ist eine Einrichtung bekannt, bei welcher anstelle einer Kamera eine über dem Probanden angebrachte Anordnung von Fotozellen zur Aufnahme der Leuchtspuren eingesetzt wird. Durch diese Anordnung entfällt der tote Winkel. Die Leuchtspuren werden dort mittels eines Digital-Computers hinsichtlich einer Berechnung der für die Cranio-Corpo-Grafie relevanten Bewegungsabweichungen analysiert. Eine Auswertung im Hinblick auf eine Interpretation erfasster Bewegungsmuster, insbesondere des Halsbewegungsmusters, ist dort jedoch nicht vorgesehen.

In dem Deutschen Gebrauchsmuster DE 295 06 404 U1 ist eine Vorrichtung zur Bestimmung der Kopfbeweglichkeit beschrieben. Dazu sind eine am Kopf des Probanden befestigte Halterung mit drei Schallsendern oder -empfängern und weitere drei Schallsender oder -empfänger als Referenzmarker am Körper des Probanden, insbesondere an der Schulter, vorgesehen, wobei die Laufzeit von Ultraschallsignalen zwischen den vom Probanden mitbewegten Markern und stationären Schallempfängern bzw. - sendern gemessen wird. Da erkanntermaßen die Bewegungen des Oberkörpers des Probanden in die Meßergebnisse der Kopfbewegung mit eingehen, kann in nicht näher beschriebener Weise die Kopfpositionen zu den vom Körper mitbewegten Referenzmarkern in Bezug gesetzt werden.

Der Erfindung liegt daher die Aufgabe zugrunde, eine Vorrichtung zur Ermittlung des Halsbewegungsmusters eines Probanden anzugeben, mit dem eine im Vergleich zum Stand der Technik besonders zuverlässige Erfassung der Halsbewegung oder Halsbeweglichkeit des Probanden möglich ist. Insbesondere soll mittels des Verfahrens ein HWS-Schleudertrauma beweisend aufgezeigt werden können.

Diese Aufgabe wird erfindungsgemäß gelöst durch die Merkmale des Anspruchs 1. Durch die erfindungsgemäße Vorrichtung wird aufbauend auf der Cranio-Corpo-Grafie aus der berührungslos erfassten Kopf-Körper-Bewegung die Halsbewegung isoliert, indem aus den erfassten Ortskurven der vom Probanden mitbewegten Marker die Differenz zwischen der Rumpfbewegung und der Kopfbewegung rechnerisch ermittelt wird.

Zur Analyse typischer Halsbewegungsmuster werden dazu zunächst die Ortskurven von am Kopf sowie an den Schultern angeordneten und vom Probanden mitbewegten Markierern dreidimensional und in Abhängigkeit von der Zeit aufgenommen. Anschließend wird die die Kopfbewegung repräsentierende Ortskurve von den die Schulterbewegungen und damit die Rumpfbewegung repräsentierenden Ortskurven subtrahiert (cervikale Subtraktionskinesiometrie). Dabei wird aus den Ortskurven beider Schulterbewegungen eine Mittelwertsbewegung der beiden Schultern und damit eine Bewegung des virtuellen Unterpunktes der Halswirbelsäule ermittelt. Von dieser Bewegung des virtuellen Unterpunktes der Halswirbelsäule wird die Ortskurve vorzugsweise des auf oder über der Stirn des Probanden angeordneten Markierers subtrahiert. Auch kann wiederum zunächst der Mittelwert aus den Ortskurven eines auf bzw. über der Stirn und auf oder über dem Hinterhaupt, vorzugsweise im Bereich der Fortführung des HWS-Unterstützungspunktes des Kopfes durch das Kopfsockelgelenk des Probanden angeordneten Markierers ermittelt werden. Dabei repräsentiert dieser Mittelwert die von der Mittelwertsbewegung der Schultern zu subtrahierende Kopfbewegung.

Die Erfindung geht dabei von der Überlegung aus, dass einerseits sowohl der sitzende als auch der stehende, gehende oder auf der Stelle tretende Proband Ganzkörperschwankungen ausführt, die sowohl am Rumpf und damit an den beiden Schultern als auch am Kopf dargestellt bzw. sichtbar sind. Andererseits sind alle Bewegungen, die in gleicher Weise am Kopf und am Rumpf auftreten, keine Halsbewegungen. Subtrahiert man daher die Kopfbewegungen von den Schulter-Rumpfbewegungen, so resultiert aus der Differenz ein ausschließlich im Zusammenhang mit den Halsbewegungen stehendes Bewegungsmuster, dessen zeitlicher Verlauf im dreidimensionalen Raum sowohl bezogen auf den Stirnpunkt als auch bezogen auf den Hinterhauptpunkt abbildbar oder darstellbar ist. Dieses zeitabhängige, dreidimensionale Bewegungsmuster kann dann zu Analysezwekken datentechnisch auf die verschiedenen Grundebenen, beispielsweise eines kartesischen Koordinatensystems, projiziert werden. Aus diesen dann zweidimensionalen Verläufen des Halsbewegungsmusters, beispielsweise in der xy- und/oder yz-Ebene, kann aus Abweichungen von Verläufen einer unverletzten Halswirbelsäule auf die Art, den Grad und vorteilhafterweise auch auf den Ort einer Schädigung der Halswirbelsäule geschlossen werden. Vorteilhafterweise kann auch eine Schädigung der funktionellen Bewegungsmuster des Kopfsockelgelenkes bestimmt werden.

Insbesondere die Lokalisierung einer Schädigung an der Halswirbelsäule ist für den Nachweis einer Kausalität einer Verletzung der Halswirbelsäule infolge eines durch einen Verkehrsunfall bedingten HWS-Schleudertraumas von erheblicher Bedeutung. Mit anderen Worten: Kann einerseits eine Schädigung der Halswirbelsäule exakt lokalisiert werden und kann andererseits dargelegt werden, dass Schädigungen der Halswirbelsäule infolge eines HWS-Schleudertraumas typischerweise an einer ganz bestimmte Stelle der Halswirbelsäule auftreten, so wäre die gewünschte Kausalität zuverlässig nachweisbar. Dies setzt aufgrund der nachfolgend erläuterten Überlegungen und Erkenntnisse voraus, dass die Halsbewegung zuverlässig von Kopf- und Rumpfbewegungen isoliert wird, um ausschließlich typische Halsbewegungen oder Halsbewegungsmuster im Hinblick auf die nachzuweisende Kausalität analysieren zu können.

Die menschliche Wirbelsäule oder das Rückgrat ist das Achsenskelett, das bekanntlich von sieben Halswirbeln, zwölf Brustwirbeln, fünf Lendenwirbeln, fünf Kreuzbeinwirbeln und fünf Steißbeinwirbeln gebildet wird. Diese Wirbel sind durch Gelenke, Bandscheiben und Bänder zu einer federnden Achsenstabkonstruktion miteinander verbunden. Dabei stellt die Halswirbelsäule (HWS) den oberen und beweglichsten Teil der gesamten Wirbelsäule mit einer physiologischen Vorwärtsbiegung bzw. Lordose dar. Die sieben Wirbel der Halswirbelsäule werden üblicherweise mit C1 bis C7 bezeichnet, wobei C1 der auch mit Atlas bezeichnete oberste Wirbel ist, unter dem der auch mit Axis bezeichnete Wirbel C2 liegt. Die darunterliegenden weiteren Halswirbel C3 bis C7 sind gegeneinander abgegrenzt und durch Bandscheiben miteinander verbunden, während zwischen der Schädelbasis und den beiden oberen Halswirbeln (C0/C1,C1/C2) derartige Bandscheiben fehlen.

Der erste Halswirbel C1 ist wirbelkörperlos und besteht aus zwei Bogen mit je einer seitlichen gelenkflächentragenden Massa laterales und je einem kurzen Querfortsatz mit relativ großen Querfortsatzlöchern. In den vorderen Teil der Bogengangslichtung ragt ein den Kopfaufsatz drehend mit der Wirbelsäule verspannender Zapfen, der sogenannte Dens axis. Der zweite Halswirbel C2 (Axis oder Epistropheus) stellt die Drehachse für den ersten Halswirbel C1 (Atlas) einschließlich des Kopfes dar. Er umfasst einen stiftartig aus dem Wirbelkörper atlaswärts ragenden Fortsatz, den sogenannten Dens, und je zwei seitliche obere und untere Gelenkflächen.

Für die Orientierung des Kopfes und des Blickes sowohl beim sitzenden, als auch beim stehenden Probanden ist eine vom menschlichen Gehirn leicht steuerbare Kopf-Halsbeweglichkeit notwendig. Diesen Bewegungen des Kopfes dienen in erster Linie die drei Kopfsockelgelenke. Das erste und obere Kopfgelenk (Articulatio atlanto occipitalis) ist ein paariges Gelenk mit elliptisch geformten Gelenkkörpern zwischen den Gelenkhöckern des auch als C0 bezeichneten Hinterhauptbeines und den oberen seitlichen Gelenkflächen des Atlas (C1). Dieses Gelenk wird vorwiegend für die Nickbewegungen, die nach vorne schiebenden Kopfbewegungen und die Seitwärtsneigung des Kopfes benutzt. Das zweite, untere Kopfgelenk (Articulatio atlanto axialis) setzt sich wiederum aus zwei, jedoch verschiedenen Gelenkeinheiten zusammen. Es ist somit ein Mehrfachgelenk zwischen dem ersten und zweiten Halswirbelkörper. Während eine der beiden Gelenkeinheiten (Articulatio atlanto axialis mediana) die Kopfdrehung übernimmt, findet in der zweiten Gelenkeinheit (Articulatio atlanto lateralis) ein Gleitkippen des Kopfes statt.

Die Halswirbelsäule (HWS) kann somit als Einheit betrachtet werden, zerfällt aber anatomisch bedingt in zwei Bewegungsstrukturen, nämlich das Kopfsockelgelenk mit den bandscheibenlosen Gleit- und Drehbewegungssegmenten C0,C1 und C2 sowie den weiteren Segmentverbindungen nach Art eines Federstabes mit bewegungsbegrenzenden Bandscheiben zwischen den Wirbelkörpern C2 bis C7 und darüber hinaus bis Th1.

Die für die Orientierung des Kopfes im Raum wichtige Wendung zur Seite oder nach vorn bzw. nach hinten kann bei versteiftem oder verletztem Hals aber auch von anderen Abschnitten der Wirbelsäule, z. B. von der Brust- und Lendenwirbelsäule, oder von den Hüftgelenken und von den unteren Extremitäten her übernommen werden. Eine tatsächlich vom Kopf stattfindende und dort erfaßte Vorwärts-, Seitwärts- oder Rückwärtsbewegung oder eine Drehbewegung kann somit auch von tieferen Regelgliedern des Körpers übernommen worden sein. Mit anderen Worten: Zum Nachweis der ursächlichen Verletzung des Halsbewegungsapparates im Bereich des Kopfsockelgelenkes ist stets zu berücksichtigen, dass der betroffene Proband gleichmäßig zu steuernde Kopfbewegungen nicht nur vom Hals aus, sondern unter Zuhilfenahme des gesamten Körpers, der Hüftgelenke, der Gelenke an den Beinen, wie auch der Brust- und Lendenwirbelsäule quasi in einem System einer zweiten und dritten Schleife bewirken kann. Für die Bestimmung einer tatsächlichen im Kopfsockelgelenk und in der Halswirbelsäule wirksamen Schädigung müssen daher die Halsbewegungen von den Bewegungen des übrigen Körpers isoliert werden.

Mittels des durch die erfindungsgemäße Vorrichtung ausgeführten, auf der Grundlage der Cranio-Corpo-Grafie weiterentwickelten Verfahrens zur Ermittlung der Halsbewegung eines Probanden, bei dem mittels an den beiden Schultern und am Kopf des Probanden angeordneten Markierern aufgenommene Ortskurven zur Isolierung der Halsbewegung in der beschriebenen Weise voneinander subtrahiert werden, können zuverlässig ausschließlich am Hals hervorgerufene Bewegungsstörungen meßtechnisch erfasst und datentechnisch ausgewertet werden. Eine weitere Differenzierung stützt sich auf die Bewegungssubtraktion des Hinterhauptpunktes (Occipitalpol) von dem Rumpfbewegungsmuster im Vergleich zur Bewegungssubtraktion des Vorderkopfpunktes (Frontalmarker) von dem Rumpfbewegungsmuster. Die Differenzierung dieser beiden Ortskurven gibt einen Aufschluß über die Störung des Kopfsockelgelenkes. Zudem können von seitlich über den Ohren oben auf dem Kopf angebrachten Markern erfasste Ortskurven zusätzlich zur differentiellen Querschwankungsanalyse herangezogen werden.

Bei einer entsprechenden Analyse der ermittelten Halsbewegungsmuster können zweckmäßigerweise Referenzmuster herangezogen werden, die in entsprechender Weise an unverletzten Probanden und an Probanden aufgenommen werden, die beispielsweise infolge eines operativen Eingriffs nachweislich eine lokalisierte Schädigung eines bestimmten Bereichs der Halswirbelsäule aufweisen. Dabei kann aufgrund der oben beschriebenen Überlegungen und Erkenntnisse davon ausgegangen werden, dass die ermittelten Halsbewegungsmuster stets durch das Kopfsockelgelenk mit seinen drei Gelenkanteilen bestimmt worden sind.

Die ermittelten Halsbewegungsmuster geben somit eine Gleit-Nick-Bewegung nach vorne durch das obere Kopfgelenk, eine Drehbewegung durch das mittlere untere Kopfgelenk und eine Seitwärtsneigung und -kippung durch das seitliche untere Kopfgelenk wieder, wobei diese Bewegung auch durch das obere Kopfgelenk unterstützt wird. Wie ein biegsamer Stab fügt der darunterliegende Halswirbelsäulenabschnitt zwischen dem dritten und siebten Halswirbel (C3 bzw. C7) noch weitere Adjustierungen in den drei Raumebenen hinzu. Diese sind jedoch winkelmäßig stark begrenzt, da diese Wirbel durch Bandscheiben miteinander verbunden sind. Auf der Wirbelebene C0/C1, d. h. im Bereich zwischen der Unterseite des Schädels und dem ersten Halswirbel C1 (Atlas), und auf der Wirbelebene C1/C2, d. h. im Bereich zwischen der Unterseite des ersten Halswirbels C1 (Atlas) und der Oberseite des zweiten Halswirbels C2 (Axis), sind keine Bandscheiben, so dass dieses Gelenk in besonderer Weise beweglich ist.

Nach diesem Verfahren durchgeführte Untersuchungen haben gezeigt, dass bei Patienten bei denen dieser Gelenkapparat beschädigt ist, typische Bewegungsveränderungen in den drehenden, schiebenden und kippenden Bewegungen in Abhängigkeit von der Verletzungsart der Bänder und Gelenke zuverlässig dargestellt werden können. Diese Erkenntnisse konnten dadurch untermauert werden, dass bei den untersuchten Patienten mit Hilfe einer kernspintomographischen Röntgenaufnahmetechnik entsprechende, typische Gelenkverletzungen bildgebend dargestellt wurden. Diese Verletzungen wurden zusätzlich durch neurochirurgische Inspektionen des Gelenks nach operativer Eröffnung verifiziert.

Das auf der Cranio-Corpo-Grafie aufbauende Verfahren hat den erheblichen Vorteil, quasi nach Art eines Lakmus-Tests bestimmte Verletzungen nach einem HWS-Schleudertrauma auch im funktionellen Bereich kausal und nachvollziehbar darzustellen, wobei das ermittelte Halsbewegungsmuster als Maß für die Beweglichkeit der Halswirbelsäule und vorzugsweise für die Bewegungsveränderungen im Kopfsockelgelenk herangezogen wird. Dabei ist gewährleistet, dass in dem ermittelten Halsbewegungsmuster keine Bewegungen oder Bewegungsanteile des übrigen Körpers, d. h. des Kopfes und/oder des Rumpfes, enthalten sind, die das Meß- oder Untersuchungsergebnis verfälschen könnten. Das erfindungsgemäße Verfahren eignet sich daher besonders für die Aufdeckung der genannten Störungen bei den sogenannten Funktionsverletzungen, bei denen bisher nur ein Verdacht auf eine Schädigung der Halswirbelsäule durch das HWS-Schleudertrauma bestand.

In besonders zweckmäßiger Ausgestaltung erfolgt die Differenzbildung zwischen der Schulter- und damit der Rumpfbewegung sowie der Kopfbewegung in jeder der drei Raumkoordinaten, wobei jeweils ein zweidimensionales Bewegungsmuster in den verschiedenen Grundebenen des kartesischen Koordinatensystems generiert wird. Zusätzlich wird vorzugsweise aus einer Projektion der die Bewegung mindestens einer Schulter und des Kopfes des Probanden repräsentierenden Ortskurven auf mindestens eine Grundebene eine Anzahl von einem Schwankungszyklus der Körperschwankung entsprechende Sequenzen ermittelt und als analysier- und typisierbares Bewegungsmuster der entsprechenden Schulter - und damit des Rumpfes - bzw. des Kopfes des Probanden hinterlegt.

Die Projektionen der oder jeder Ortskurve auf die Grundebenen des Koordinatensystems werden dazu zweckmäßigerweise aus dem Datensatz ermittelt. Dazu werden vorteilhafterweise die Ortskurven jeweils als Datenfeld (zeitabhängig) hinterlegt.

Vorteilhafte Weiterbildungen und Ausgestaltungen sind Gegenstand der Unteransprüche.

So weist die Vorrichtung eine mit einer Empfängeranordnung zur Aufnahme der Ortskurve jedes Markierers verbundene Datenverarbeitungsanlage auf, die eine Verarbeitungsstufe zur Berechnung des die Ortskurven repräsentierenden Datensatzes aus den Signalen der Empfängeranordnung umfasst. Dieser Verarbeitungsstufe ist eine Subtraktionsstufe nachgeordnet, die anhand des Datensatzes die Differenz zwischen dem Mittelwert der beiden die Schulterbewegung repräsentierenden Ortskurven und der die Kopfbewegung repräsentierenden Ortskurve bildet sowie in der oder jeder der drei Raumkoordinaten den Verlauf des daraus abgeleiteten Halsbewegungsmusters generiert.

Die Empfängeranordnung umfasst zweckmäßigerweise zwei zueinander rechtwinklig angeordnete Empfänger. Die Empfänger können im Raum verteilt zueinander angeordnete Ultraschallsende- und -Empfangsgeräte, CCD-Kameras (Video-Kameras), Fotoelemente oder dgl. zur Aufnahme und ggf. Vorverarbeitung von akustischen oder optischen Signalen sein. Durch die rechtwinklige Anordnung der Empfänger zueinander erfolgt eine Aufnahme der Ortskurven der Markierer in zumindest zwei unterschiedlichen Ebenen, z. B. in der xy-Ebene und in der yz-Ebene oder in der xz-Ebene. Die Koordinaten der Ortskurven bezüglich der dritten Ebene können dann aus den Messdaten der beiden Empfänger berechnet werden. Bei Verwendung von Ultraschall anstelle von Licht zur Markierung kann die Messung auch in einem nicht abgedunkelten Raum ausgeführt werden.

Zur Hinterlegung der in einer aktuellen Messung erfassten Daten ist der Verarbeitungsstufe eine Datenbank nachgeordnet, in der vorzugsweise auch in einer Vielzahl von Referenzmessungen ermittelte Referenzdatensätze hinterlegt werden. Ein Analysemodul oder eine Analysestufe der Datenverarbeitungsanlage kann dann aus dem aktuell erfaßten Datensatz und aus dem entsprechenden Referenzdatensatz eine Anzahl von Kenngrößen bzw. Referenzgrößen ermitteln, die in einem Vergleichsmodul oder in einer Vergleichsstufe anhand der Kenn- und Referenzgrößen in der Art einer Mustererkennung den Grad der Übereinstimmung zwischen den Datensätzen ermittelt. Die Datenverarbeitungsanlage kann dann anschließend jedem Datensatz eine einer Verletzung oder Schädigung entsprechende Kennung zuordnen und den Datensatz anhand der Kennung der Datenbank zur Erweiterung des entsprechenden Referenzdatensatzes übergeben.

Die Verarbeitungsstufe ordnet die Ortskurve jedes Markierers zweckmäßigerweise als Datenfeld dem Datensatz zu. Dadurch ist eine Matrix mit einer der Anzahl der Markierer entsprechenden Anzahl von Datenfeldern gegeben, wobei jedes Datenfeld die drei Ortskoordinaten bezogen auf ein kartesisches Koordinatensystem zum jeweiligen Zeitpunkt enthält. Der Verarbeitungsstufe ist vorteilhafterweise ein temporärer Datensatzspeicher zur Zwischenspeicherung der erfaßten Meßdaten nachgeordnet.

Die mit der Erfindung erzielten Vorteile bestehen insbesondere darin, daß durch eine rechnergestützte Auswertung der bei einer Anzahl von optisch oder akustisch erfaßten Bewegungen erfaßten Meßdaten und der daraus durch Differenzbildung der Schulter- und Kopfbewegungen abgeleiteten Halsbewegungsmuster anhand entsprechender Kurvenverläufe eine zuverlässige Aussage über den Grad, das Ausmaß und den Ort einer Verletzung oder Schädigung der Halswirbelsäule eines Probanden getroffen werden kann. Während die Meßdatenerfassung praktisch berührungsfrei am Körper des Probanden erfolgt, erfolgt die Auswertung in einer vom Körper des Probanden losgelösten Datenverarbeitungsanlage, in der außerhalb des Körpers die Meßdaten verarbeitet und für eine Analyse der Halsbewegungsmuster aufbereitet werden. In entsprechenden Darstellungen der Ortskurven ist zudem zwischen summarischen Kopf-Hals-Bewegungen gegenüber den Körper- und Rumpfbewegungen sowie zwischen Kopfbewegungen (Drehen, Nikken) gegenüber Halsbewegungen unterscheidbar.

Die Auswertung der Halsbewegungsmuster sowohl gesunder als auch krankhafter HWS-Bewegungen ermöglicht einerseits das Anlegen einer Wissensbasis mit einer Vielzahl von Referenzdaten und -mustern, anhand derer aktuell erfaßte und nicht diagnostizierte Halsbewegungsmuster bekannten Verletzungen und Schädigungsarten zugeordnet werden können. Andererseits können dadurch und anhand der Halsbewegungsmuster selbst qualitative und quantitative sowie insbesondere nachvollziehbare Aussagen über einen möglichen kausalen Zusammenhang zwischen den Störungen gemacht und als Nachweis oder Beweismittel für ein insbesondere unfallbedingtes HWS-Schleudertrauma herangezogen werden.

Ein Ausführungsbeispiel der Erfindung wird nachfolgend anhand einer Zeichnung näher beschrieben. Darin zeigen:

Fig. 1        schematisch eine Vorrichtung mit zur Auswertung eines Bewegungsmusters vorgesehenen Komponenten,

Fig. 2 bis 5    Kopf- und Schulterbewegungsmuster in einer Projektion auf die yz-Ebene (linke obere bzw. untere Figurenhälfte) und auf die xy-Ebene (rechte obere bzw. untere Figurenhälfte),

Fig. 6 bis 9    in einem Amplituden-Zeit-Schema Differenzbewegungsmuster der den Bewegungsmustern gemäß den Figuren 2 bis 6 entsprechenden Halsbewegungen in den drei Raumkoordinaten x,y,z, und

Fig. 10      in einer Darstellung gemäß den Figuren 6 bis 9 ein weiteres Halsbewegungsmuster.

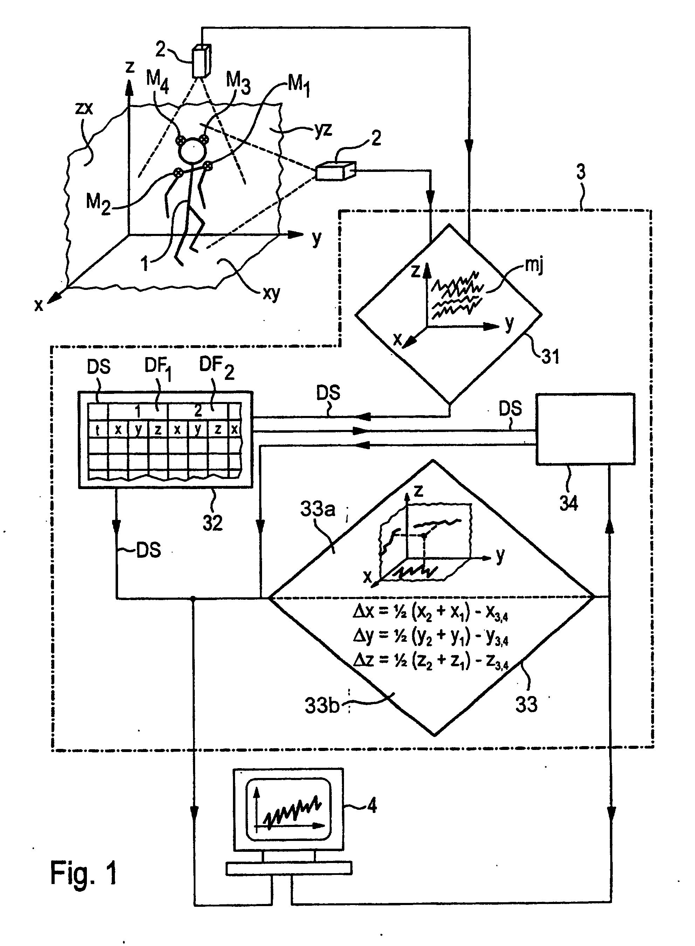

Zur Erfassung des Kopf-Körper-Bewegungsmusters eines Probanden 1 sind gemäß Fig. 1 zwei zueinander rechtwinklig ausgerichtete Empfänger 2 vorgesehen. Diese empfangen nicht näher dargestellte Signale von einer Anzahl von mit dem Probanden 1 mitbewegten Markierern $M_i$. Die Visualisierung der Körperbewegung kann besonders einfach auf optischem Wege realisiert werden. In diesem Fall verwendet man Glühlampen oder Leuchtdioden als Markierer $M_i$ und entsprechend je eine Kamera, wie z.B. eine Videokamera, als Empfänger 2. Eine Markierung des Bewegungsmusters ist auch mittels Ultraschallsendern als Markierer $M_i$ und Ultraschallempfängern als Empfänger 2 möglich. Alternativ können auch passive Markierer $M_i$ eingesetzt werden, die das von einer externen Quelle emittierte Signal lediglich reflektieren. Wie in der sogenannten Cranio-Corpo-Graphie (CCG oder UCCG) üblich, beschränkt sich dabei die Beobachtung zweckmäßigerweise auf die Kopf- und Schulterbewegung des Probanden 1. Dazu wird je ein Markierer $M_1$ und $M_2$ auf oder über der linken bzw. auf oder über der rechten Schulter des Probanden 1 sowie je ein weiterer Markierer $M_3$ und $M_4$ auf oder über dessen Stirn bzw. auf oder über dessen Hinterhaupt angebracht.

Die Empfänger 2 führen je ein zweidimensionales Bild der Bewegung der Markierer $M_i$ einer in einer Datenverarbeitungsanlage 3 enthaltenen Verarbeitungsstufe 31 zu, die aus den von den Empfängern 2 übermittelten Bildern die Ortskurve $m_i$ jedes Markierers $M_i$ im dreidimensionalen Raum in Abhängigkeit von der Zeit t ermitteln. Die Raumkoordinaten jeder Ortskurve $m_i$ werden in einem kartesischen Koordinatensystem x, y,z dargestellt, wobei die Ausgangsstellung des Probanden 1 dem Null-Punkt zugeordnet ist und damit die x-Achse der Lateralachse entspricht. Die y-Achse verläuft dann horizontal in Laufrichtung des Probanden 1, während sich die z-Achse vertikal nach oben erstreckt. Die von den jeweiligen Achsen gebildeten Grundebenen des Koordinatensystem sind die xy-Ebene (horizontal), die yz-Ebene (longitudinal vertikal) und die zx-Ebene (lateral vertikal).

Die Berechnung der Ortskurve $m_i$ jedes Markierers $M_i$ erfolgt mittels der Datenverarbeitungsanlage 3 anhand eines Algorithmus der Verarbeitungsstufe 31. Falls für die Empfänger 2 eine analoge Aufzeichnungstechnik verwendet wird, findet in der Verarbeitungsstufe 31 zunächst eine Umwandlung von analogen Daten in digitale Daten statt. Die Verarbeitungsstufe 31 übergibt die Ortskurven $m_i$ als Datensatz DS einem vorzugsweise temporären Datensatzspeicher 32. Dieser ist dabei in Datenfelder $DF_i$ gegliedert, wobei ein Datenfeld $DF_i$ die Ortskurve $m_i$ eines Markierers $M_i$ repräsentiert.

Der Datensatzspeicher 32 stellt den Datensatz DS einem in Form von Software realisierten Analysemodul 33 mit einer Abbildungsstufe 33a und mit einer Subtraktionsstufe 33b zur Verfügung. Außerdem wird der Datensatz DS in einer Datenbank 34 hinterlegt. Die Abbildungsstufe 33a stellt durch Datenauswahl aus dem Datensatz DS jeweils eine Projektion der Ortskurven $m_i$ auf die Grundebenen xy,yz und zx her. Da die Ortskurven $m_i$ typischerweise eine durch eine Körperschwankung hervorgerufene periodische Struktur aufweisen, führt ein Algorithmus des Analysemoduls 33 eine Unterteilung der Ortskurven $m_i$ in periodische Sequenzen durch. Eine derartige Sequenz, deren Anfang und Ende jeweils durch eine scharfe Richtungsänderung der Ortskurve $m_i$ gekennzeichnet ist, entspricht dabei genau einem Zyklus der Körperschwankung. Aus den auf die Grundebenen xy (horizontale Ebene), yz (longitudinal-vertikale Ebene) und zx (lateral-vertikale Ebene) projizierten und in Sequenzen unterteilten Ortskurven $m_i$ können in nicht näher dargestellter Art und Weise weitere Algorithmen des Analysemoduls 33 eine Anzahl von Kenngrößen oder Parametern ableiten.

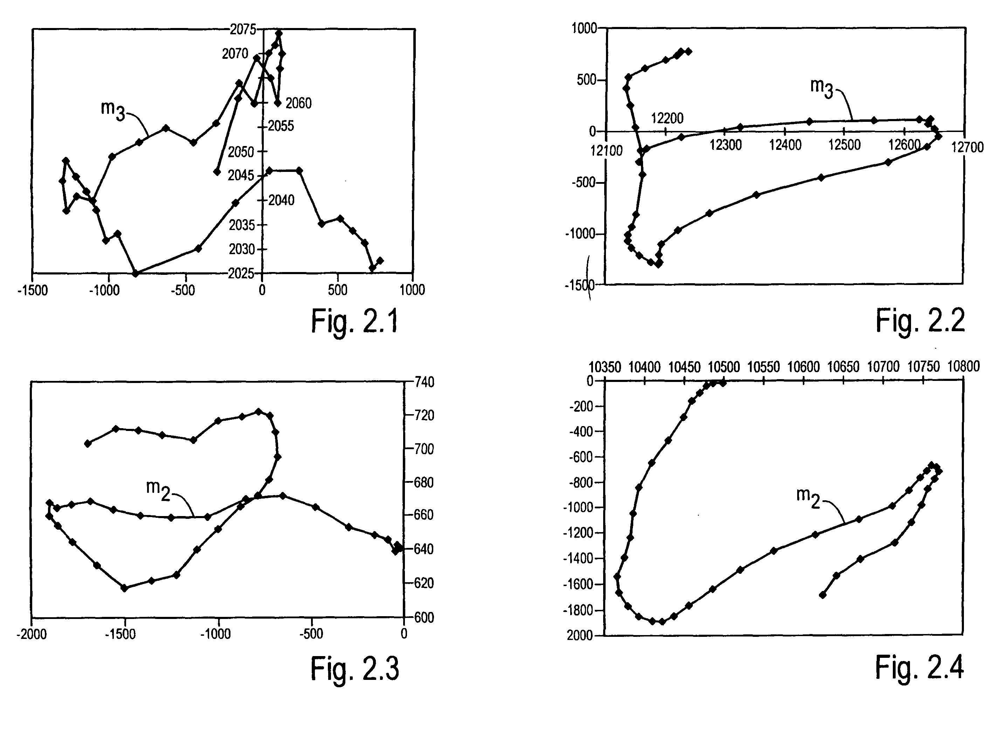

Für die Auswertung typischer Schulter- und Kopf-Bewegungsmuster werden vorzugsweise die Projektionen der Ortskurven $m_i$ auf die xy-Ebene und auf die yz-Ebene geometrisch und physikalisch vermessen. Entsprechende Bewegungsmuster sind in den Figuren 2 bis 5 dargestellt. Diese zeigen jeweils in der oberen Figurenhälfte ein bei einer Schrittfolge des jeweiligen Probanden 1 erzeugtes Stirnpunkt-Bewegungsmuster und in der jeweils unteren Figurenhälfte die zugehörigen Bewegungsmuster der rechten Schulter. Die linke Figurenhälfte entspricht dabei jeweils der yz-Ebene, während die rechte Figurenhälfte jeweils eine Darstellung in der xy-Ebene zeigt.

Aufgrund der periodischen Struktur jeder Ortskurve $m_i$ sind die Amplitude, die Periodendauer, die Frequenz einer Schwankung sowie die während einer Schwankungsperiode in und quer zur Schwankungsrichtung zurückgelegte Strecke (Schrittweite) relevante Parameter. Diese lassen sich sowohl aus einer einzelnen Sequenz ermitteln (single-step-Analyse) als auch aus einer Anzahl von Sequenzen statistisch ableiten und in Form von Mittelwert und Standardabweichung angeben (whole-reaction-Analyse). Des weiteren können Unregelmäßigkeiten in der Körperschwankung durch Angabe einer Amplitudenverteilung und einer mittels Spektralanalyse (Fourriertransformation) gewonnenen Frequenzverteilung quantifiziert werden. Ferner können aus der Schwerpunktsbewegung des Körpers, der Drehung des Körpers gegenüber dem Raum sowie der Drehung des Kopfes relativ zum Rumpf physikalische Parameter und Kenngrößen ermittelt werden. Dazu können die Ortskurven $m_i$ mehrerer Markierer $M_i$ auch miteinander kombiniert werden.

Die Subtraktionsstufe 33b bildet durch Auswahl aus dem Datensatz DS die Differenz zwischen der durch die entsprechenden Ortskurven $m_i$ repräsentierten Schulterbewegung und der zeitgleich erfaßten Kopfbewegung. Dabei wird zunächst der Mittelwert aus den erfaßten Ortskurven $m_2$ und $m_1$ der rechten bzw. linken Schulter-Markierer $M_2$ bzw. $M_3$ gebildet. Dieser Mittelwert-Verlauf der Schulter- und damit der Rumpfbewegung repräsentiert somit den virtuellen Unterpunkt der Halswirbelsäule des Probanden 1. Durch Subtraktion der Kopfbewegung von diesem Mittelwert ergibt sich die somit von Kopf- und Schulter- oder Rumpfbewegungen isolierte Halsbewegung oder Beweglichkeit der Halswirbelsäule des Probanden 1. Dazu kann lediglich z. B. die Ortskurve $m_3$ des Stirn-Markierers $M_3$ herangezogen werden. Alternativ kann die Kopfbewegung durch Mittelwertbildung der Ortskurven $m_3$ und $m_4$ der Stirn- bzw.

Hinterhauptmarkierer $M_3$ bzw. $M_4$ von der Subtraktions-stufe 33b berechnet werden. Die Differenzbildung und damit die Isolierung des Halsbewegungsmusters aus der erfaßten Kopf-Körper-Bewegung (Cranio-Corpo-Gramm) erfolgt für jede der drei Raumkoordinaten gemäß den Beziehungen:

$$\Delta x = \tfrac{1}{2}(x_2 + x_1) - x_{3,4} = \tfrac{1}{2}(x_{rS} + x_{lS}) - x_K$$

$$\Delta y = \tfrac{1}{2}(y_2 + y_1) - y_{3,4} == \tfrac{1}{2}(y_{rS} + y_{lS}) - y_K$$

$$\Delta z = \tfrac{1}{2}(z_2 + z_1) - z_{3,4} = \tfrac{1}{2}(z_{rS} + z_{lS}) - z_K$$

Dabei repräsentiert der jeweilige Minuend den Verlauf des Mittelwertes der Schulterbewegungen (rechte Schulter rS, linke Schulter lS) und damit den Verlauf des virtuellen Unterpunktes der Halswirbelsäule, während der Subtrahend den Verlauf der Kopfbewegung (Kopf K), z. B. ebenfalls als Mittelwert, charakterisiert.

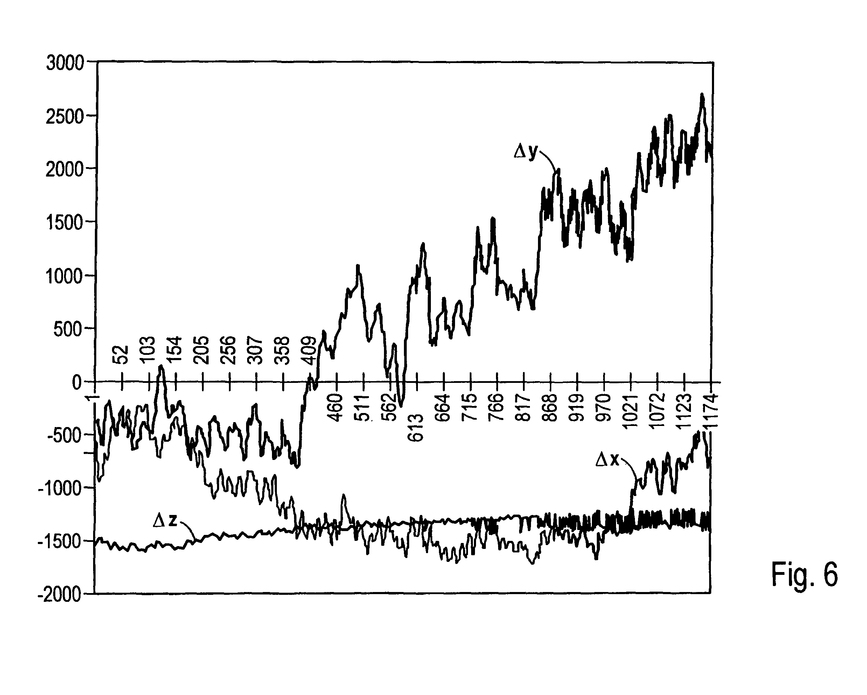

Die entsprechenden Verläufe der Halsbewegungsmuster $\Delta x, \Delta y, \Delta z$ in den drei Raumkoordinaten zeigen die Figuren 6 bis 9, wobei $\Delta x$ das laterale, $\Delta y$ das Anterior-Posterior- und $\Delta z$ das vertikale/cervikale Halsbewegungsmuster repräsentiert. Die durch das beschriebene Subtraktionsverfahren (cervikale Subtraktionskinesiometrie) ermittelten Halsbewegungsmuster der Figuren 6 bis 9 sind aus den den Figuren 2 bis 5 zugrundeliegenden Ortskurven $m_i$ und Datensätzen DS ermittelt worden.

Die Figuren 2 und 6 zeigen räumlich/zeitliche Messdiagramme von 3D-Messungen der Ortskurven $m_i$ der an der Stirn erfassten Kopfbewegungen (Fig. 2 oben) und der Bewegung der rechten Schulter (Fig. 2 unten) bzw. daraus abgeleitete Halsbewegungen in drei Richtungs-Zeit-Ebenen eines 54-jährigen Mannes nach einem Kopf-Hals-Schleudertrauma. Dabei repräsentieren das linke obere und untere Diagramm in Fig. 2 jeweils eine Projektion der Ortskurve $m_3$ bzw. $m_2$ auf die yz-Ebene, während das rechte obere und untere Diagramm eine Projektion der jeweiligen Ortskurve $m_3$ bzw. $m_2$ auf die yz-Ebene darstellen.

Der Verlauf der projizierten Ortskurve $m_2$ in der rechten unteren Figurenhälfte der Fig. 2 zeigt in der entsprechenden xy-Bewegungsanalyse der rechten Schulter normale Schrittzyklen von ca. 2¼ Pi als Ausdruck der mittels der Kreisfunktion dargestellten Periodizität der Schrittbewegung während zwei Sekunden. In der yz-Darstellung gemäß der linken unteren Figurenhälfte der Fig. 2 ist auffällig, dass während der Schrittzyklen gewisse vertikale Unregelmäßigkeiten auftreten. Die Differenzbildung zur Isolierung der Halsbewegung oder des Halsbewegungsmusters erfolgt nach den genannten Beziehungen für $\Delta x$, $\Delta y$, $\Delta z$.

Anhand der durch die Subtraktionsanalyse der Halsbewegung ermittelten Verläufe in der zugehörigen Figur 6 ist aus dem Amplitudenzeitbewegungsmuster $\Delta y$ ein stark disrhythmisches Halsbewegungsmuster mit gestörten Kopf-Nick- und Kopf-Schiebe-Bewegungen in der y-Koordinate festzustellen. Dieses Muster weist auf eine deutliche Störung im Kopfsockelgelenk hin. Dabei stellt die y-Koordinate die Kopf-Nick-Bewegungen, d. h. die Anterior-Posterior-Bewegungen dar, während die x-Koordinate die Lateralbewegungen und Querschwankungen repräsentiert. Die Vertikalbewegungen, d. h. die Stampfbewegungen, sind mittels der z-Koordinate dargestellt.

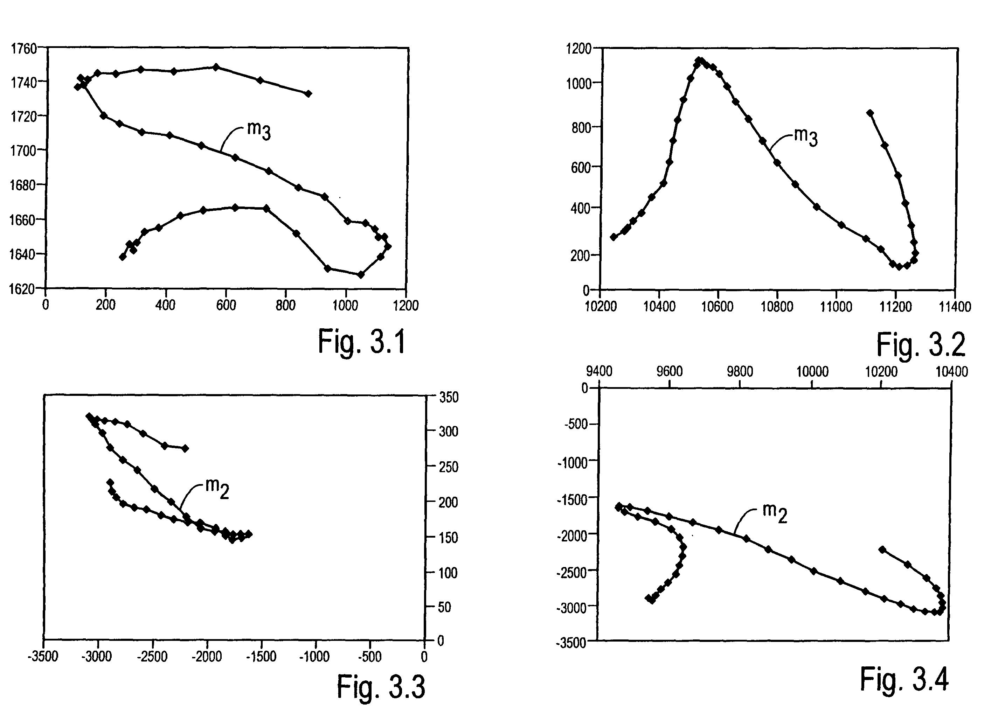

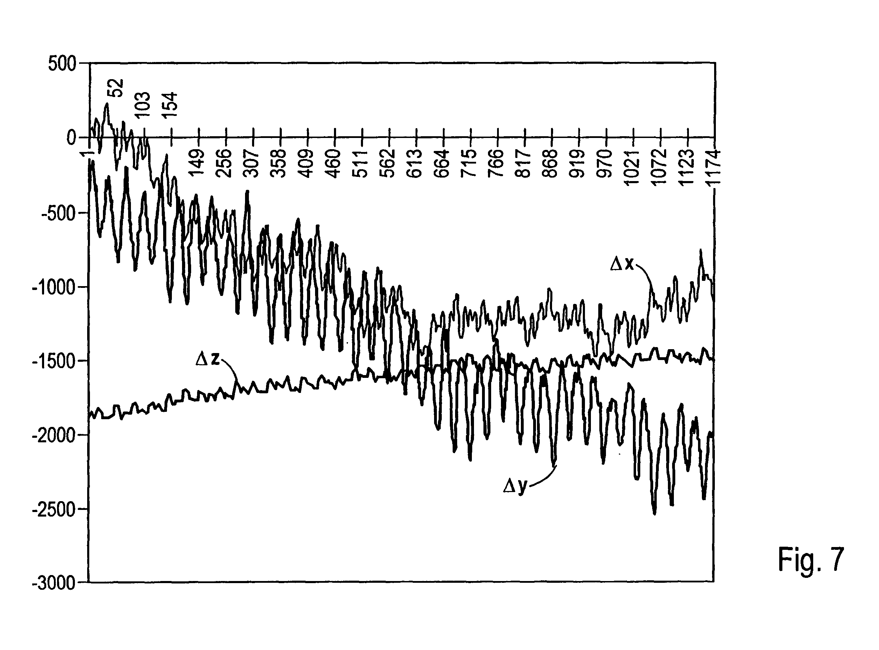

Die Figuren 3 und 7 zeigen räumlich/zeitliche Messdiagramme von 3D-Messungen der Ortskurven $m_i$ der Kopfbewegungen an der Stirn (Fig. 3 oben) und an der rechten Schulter (Fig. 3 unten) bzw. daraus abgeleitete Halsbewegungen in drei Richtungs-Zeit-Ebenen eines 60-jährigen Mannes mit zentralen Schwindelbeschwerden. Dabei repräsentieren das linke obere und untere Diagramm in Fig. 3 jeweils eine Projektion der Ortskurve $m_3$ bzw. $m_2$ auf die yz-Ebene, während das rechte obere und untere Diagramm eine Projektion der jeweiligen Ortskurve $m_3$ bzw. $m_2$ auf die yz-Ebene darstellen. Die yz- und xy-Darstellungen gemäß Fig. 3 zeigen regelrechte Schrittzyklen von 2 Pi während zwei Sekunden. Die Subtraktionsanalyse der Kopfbewegungen zeigt anhand des Differenzverlaufs $\Delta y$ nach Fig. 7 vergröberte Schwankungen in der Nickachse y. Auffällig ist das unregelmäßige Querschwankungsmuster $\Delta x$ sowie das Auf- und Absteigemuster $\Delta z$ des Kopfes in der z-Koordinate während der einzelnen Schrittzyklen.

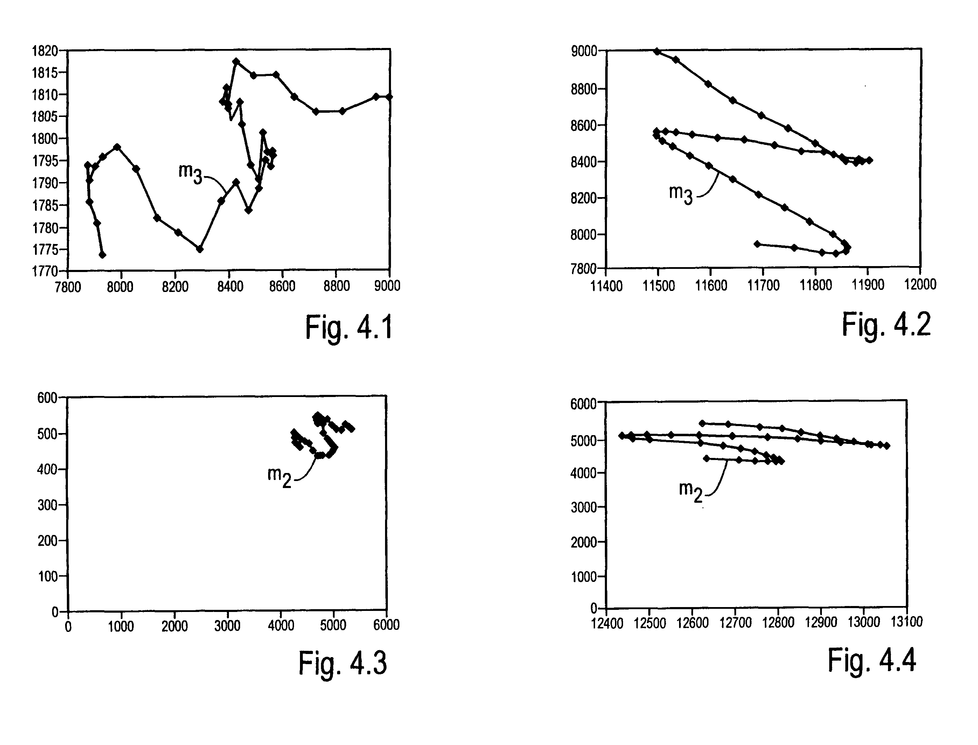

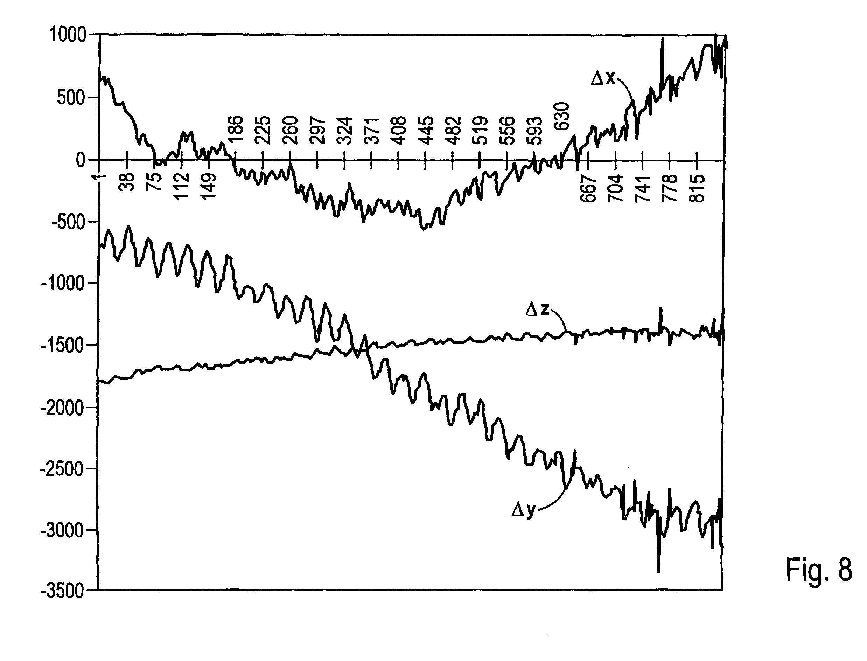

Die Figuren 4 und 8 zeigen räumlich/zeitliche Messdiagramme von 3D-Messungen der Ortskurven $m_i$ der Kopfbewegungen an der Stirn (Fig. 4 oben) und an der rechten Schulter (Fig. 4 unten) bzw. daraus abgeleitete Halsbewegungen in drei Richtungs-Zeit-Ebenen eines 63-jährigen Mannes bei kombinierten stato-akustischen Störungen, d. h. bei mehrfachen neurosensorischen Störungen des Innenohres im Hör- und Gleichgewichtsabschnitt. Dabei repräsentieren das linke obere und untere Diagramm in Fig. 4 jeweils eine Projektion der Ortskurve $m_3$ bzw. $m_2$ auf die yz-Ebene, während das rechte obere und untere Diagramm eine Projektion der jeweiligen Ortskurve $m_3$ bzw. $m_2$ auf die yz-Ebene darstellen. In der yz-Darstellung gemäß der oberen und unteren rechten Figurenhälfte der Fig. 4 ist bezüglich des Kopfes bzw. der Schulter ein irreguläres und gehemmtes Bewegungsmuster zu beobachten, während die xy-Darstellung gemäß der rechten Figurenhälfte ein regelrechtes Schrittzyklusmuster über 3 Pi zeigt. Die Subtraktionsanalyse der Halsbewegungsmuster gemäß Fig. 8 zeigt ein nur leicht gestörtes Bild der Schrittzyklen in der Kopf-Nick-Achse $\Delta y$. Die Kopfquerschwankungsachse ist irregulär und durch ein disrhythmisches Bewegungsmuster $\Delta x$ gekennzeichnet. Die Vertikal-Bewegung, d. h. das Auf- und Abwärts-Bewegungsmuster $\Delta z$, ist in der Amplitudenzeitdarstellung im Wesentlichen normal.

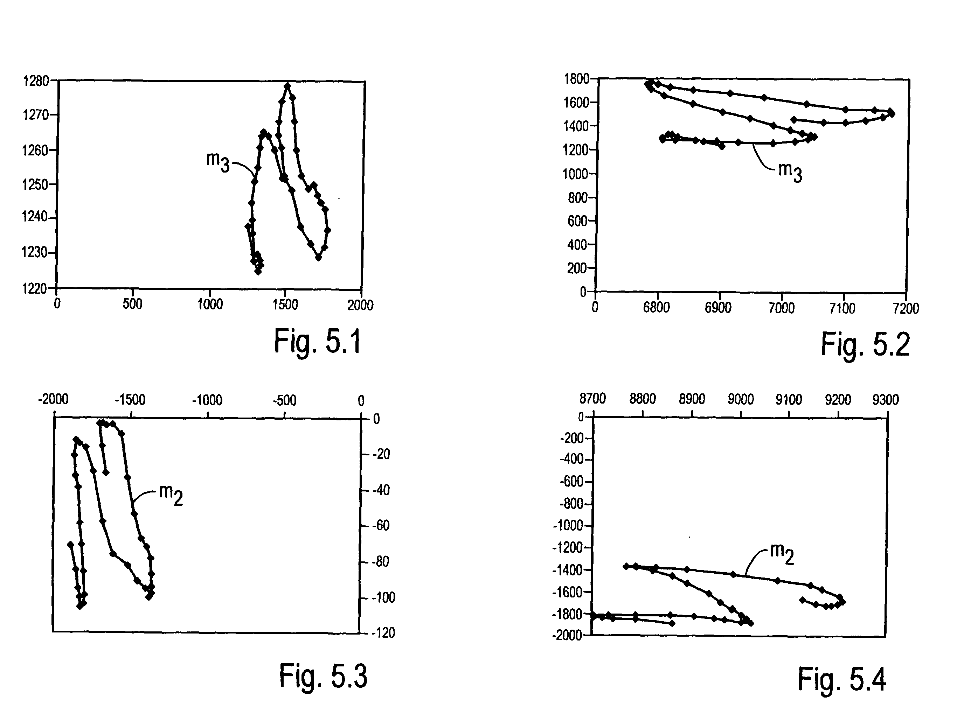

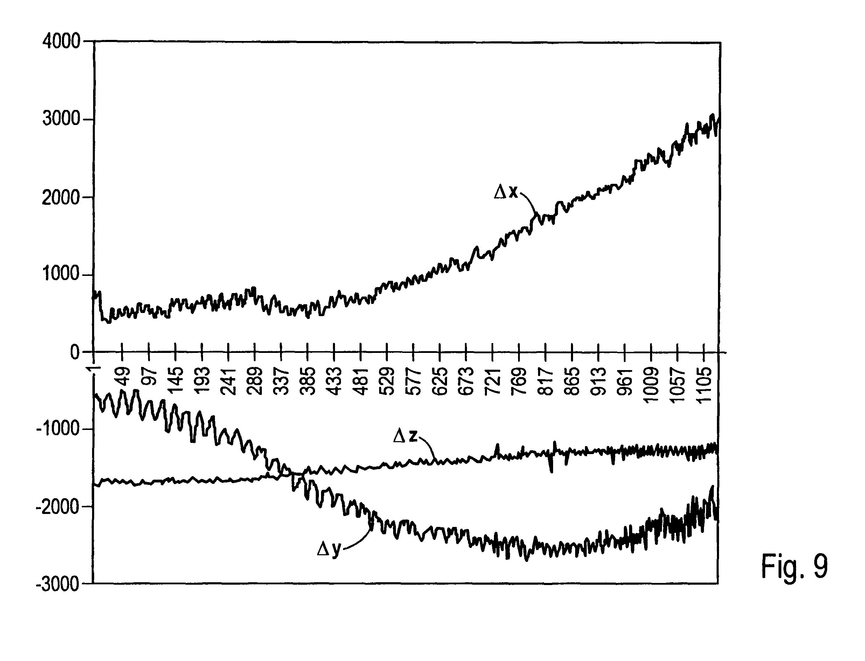

Die Figuren 5 und 9 zeigen räumlich/zeitliche Messdiagramme von 3D-Messungen der Ortskurven $m_i$ der Kopfbewegungen an der Stirn (Fig. 5 oben) und an der rechten Schulter (Fig. 5 unten) bzw. daraus abgeleitete Halsbewegungen in drei Richtungs-Zeit-Ebenen einer 25-jährigen Frau nach einem HWS-Schleudertrauma. Dabei repräsentieren das linke obere und untere Diagramm in Fig. 5 jeweils eine Projektion der Ortskurve $m_3$ bzw. $m_2$ auf die yz-Ebene, während das rechte obere und untere Diagramm eine Projektion der jeweiligen Ortskurve $m_3$ bzw. $m_2$ auf die yz-Ebene darstellen. Beim Treten auf der Stelle (Schritttest) bildet dieser Proband 1 gemäß Fig. 5 annähernd 4 Pi während zwei Sekunden ab. Die Subtraktionsanalyse der Halsbewegungen zeigt gemäß Fig. 9 ein gehemmtes Bewegungsmuster $\Delta y$ in der Kopf-Nick-Achse. Auch die Querschwankungen $\Delta x$ sind auffällig stark gehemmt. Die vertikalen Kopfbewegungen sind gemäß des $\Delta z$-Verlaufes stabil. Dieses Halsbewegungsmuster charakterisiert den sogenannten "steifen Hals" mit einer auffälligen Bewegungshemmung des Kopfes.

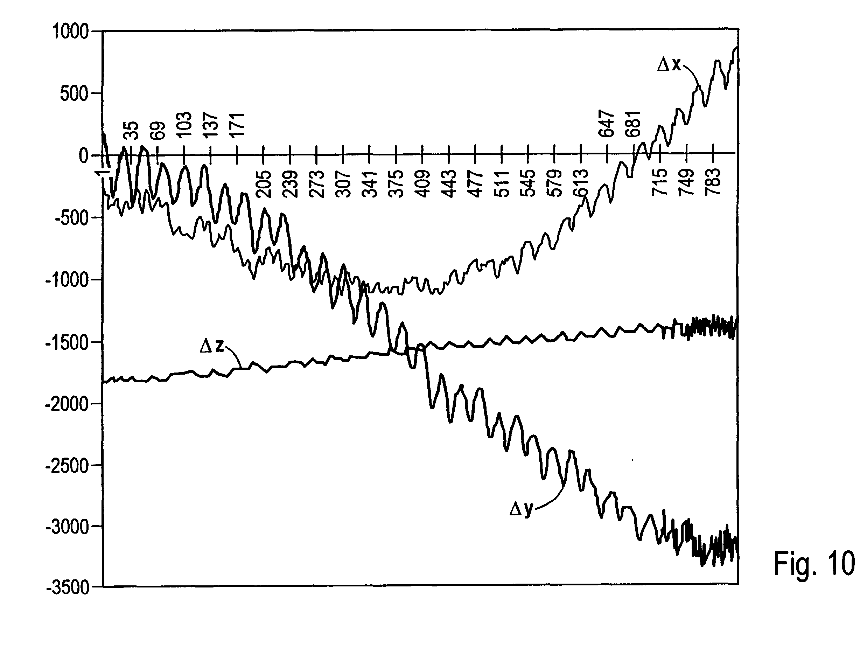

Fig. 10 zeigt in einer Darstellung gemäß den Figuren 6 bis 9 den Zustand eines 47-jährigen Mannes mit Tinnitusbeschwerde, d. h. Innenohrgeräusch-Störungen. Das Schrittzyklusmuster der Subtraktionsanalyse der Halsbewegungen zeigt ein regelmäßiges $\Delta y$-Bewegungsmuster, das in dieser Form durch Kopfanschieben, Kopfabsenken und Kopfaufrichten in besonderer Weise der Blickstabilisierung der Augen dient.

Zusätzlich zu der physikalischen und/oder geometrischen Charakterisierung kann z. B. auch die Übereinstimmung der Linienform der Ortskurven $m_i$ mit in der Datenbank 34 hinterlegten Vergleichs- oder Referenzmustern ermittelt werden. Ein entsprechender Vergleich kann auf eine einzelne Sequenz oder auf die gesamte Ortskurve $m_i$ bezogen sein. Dabei können Schwankungssequenzen anhand der Form und eventueller Umkehrbereiche charakterisiert werden. Typisch sind dabei bogen-, schleifen- oder spitzenförmige Umkehrbereiche. Zur Beschreibung der gesamten Ortskurve $m_i$ können die Umrißform der von einer Projektion einer Ortskurve $m_i$ überstrichenen Fläche herangezogen werden.

Zur Bewertung des Datensatzes DS wird dem Analysemodul 33 aus der Datenbank ein Referenzdatensatz zur Verfügung gestellt, der eine dem Datensatz DS entsprechende Datenstruktur aufweist und in einer Referenzmessung analog zur Erstellung des aktuellen Datensatzes DS erstellt wird. Die Datenverarbeitungsanlage 3 ermittelt aus dem Referenzdatensatz durch Vergleich mit dem aktuellen Datensatz DS den Grad der Übereinstimmung zwischen den Datensätzen. Anhand eines direkten oder indirekten Mustervergleichs kann dann ein aktuelles Bewegungsmuster typisiert oder zumindest qualitativ eingeordnet werden.

Das Analysemodul 33 ist zur Ausgabe der Kopf- und Schulter-Bewegungsmuster sowie der Halsbewegungsmuster mit einem Ausgabemodul 4, z. B. einem Bildschirm, einem Drucker oder einem Plotter, verbunden. Über dieses Ausgabemodul 4 kann weiterhin das im Datensatzspeicher 32 in Form des Datensatzes DS niedergelegte Bewegungsmuster ausgegeben werden.

Bezugszeichenliste

| | |
|---|---|
| 1 | Proband |
| 2 | Empfänger |
| 3 | Datenverarbeitungsanlage |
| 4 | Ausgabemodul |
| 31 | Verarbeitungsstufe |
| 32 | Datensatzspeicher |
| 33 | Analysemodul |
| 33a | Abbildungsstufe |
| 33b | Subtraktionsstufe |
| 34 | Datenbank |
| $M_i$ | Markierer |
| DS | Datensatz |
| $DF_i$ | Datenfeld |
| $m_i$ | Ortskurve |
| x | laterale Achse |
| y | longitudinale Achse |
| z | vertikale Achse |
| xy | horizontale Ebene |
| yz | longitudinal-vertikale Ebene |
| zx | lateral-vertikale Ebene |
| $\Delta x$ | laterales Halsbewegungsmuster |
| $\Delta y$ | Anterior-Posterior-Halsbewegungsmuster |
| $\Delta z$ | vertikales/cervikales Halsbewegungsmuster |

**Patentansprüche**

1. Vorrichtung zur Auswertung eines mittels einer Anzahl von an den Schultern und am Kopf eines Probanden angeordneten Markierern ($M_i$) erfaßten Bewegungsmusters, mit einer mit einer Empfängeranordnung (2) zur Aufnahme der Ortskurve ($m_i$) jedes Markierers ($M_i$) verbundenen Datenverarbeitungsanlage (3), die eine Verarbeitungsstufe (31) zur Berechnung eines die Ortskurve ($m_i$) repräsentierenden Datensatzes (DS) aus Signalen der Empfängeranordnung (2) umfaßt,

   **dadurch gekennzeichnet,**

   **dass** die Datenverarbeitungsanlage (3) ein Analysemodul (33) mit einer Subtraktionsstufe (33b) umfaßt, die dazu ausgebildet ist, anhand des Datensatzes (DS) die Differenz zwischen dem Mittelwert der beiden die Schulterbewegungen repräsentierenden Ortskurven ($m_2, m_3$) und der die Kopfbewegung repräsentierenden Ortskurve ($m_1$) zu bilden und in mindestens einer der drei Raumkoordinaten (x,y,z) den Verlauf des daraus abgeleiteten Halsbewegungsmusters ($\Delta x, \Delta y, \Delta z$) zu generieren.

**2.** Vorrichtung nach Anspruch 1,
  **dadurch gekennzeichnet,**
**daß** die Empfängeranordnung zwei zueinander rechtwinklig angeordnete Empfänger (2) umfaßt.

**3.** Vorrichtung nach Anspruch 1 oder 2,
  **dadurch gekennzeichnet,**
**daß** die Verarbeitungsstufe (31) die Ortskurve ($m_i$) jedes Markierers ($M_i$) als Datenfeld ($DF_i$) dem Datensatz (DS) zuordnet.

**4.** Vorrichtung nach einem der Ansprüche 1 bis 3,
  **dadurch gekennzeichnet,**
**daß** der Verarbeitungsstufe (31) ein temporärer Datensatzspeicher (32) nachgeordnet ist.

**5.** Vorrichtung nach einem der Ansprüche 1 bis 4,
  **dadurch gekennzeichnet,**
**daß** die Datenverarbeitungsanlage (3) den Verlauf des durch die Differenzbildung ermittelten Halsbewegungsmusters ($\Delta x,\Delta y,\Delta z$) einem Ausgabemodul (4) zur Darstellung des Bewegungsmusters zuführt.

**Claims**

**1.** Apparatus for evaluating a movement pattern acquired by means of a number of markers ($M_i$) arranged on the shoulders and on the head of a subject, having a data processing system (3) which is connected to a receiver arrangement (2) for recording the locus ($m_i$) of each marker ($M_i$) and which comprises a processing stage (31) for calculating a data record (DS), representing the locus ($m_i$), from signals of the receiver arrangement (2),
**characterized in that**
the data processing system (3) comprises an analysis module (33) with a subtraction stage (33b) which is designed, using the data record (DS), to form the difference between the mean value of the two loci ($m_2,m_3$) representing the shoulder movements and the locus ($m_1$) representing the head movement, and to generate the profile of the neck movement pattern ($\Delta x,\Delta y,\Delta z$) derived therefrom in at least one of the three space coordinates (x,y,z).

**2.** Apparatus according to Claim 1,
**characterized in that**
the receiver arrangement comprises two receivers (2) arranged at right angles to one another.

**3.** Apparatus according to Claim 1 or 2,
**characterized in that**
the processing stage (31) assigns the locus ($m_i$) of each marker ($M_i$) as data field ($DF_i$) to the data record (DS).

**4.** Apparatus according to one of Claims 1 to 3,

**characterized in that**
a temporary data record memory (32) is arranged downstream of the processing stage (31).

**5.** Apparatus according to one of Claims 1 to 4,
**characterized in that**
the data processing system (3) feeds the profile of the neck movement pattern ($\Delta x,\Delta y,\Delta z$) determined by the subtraction to an output module (4) for displaying the movement pattern.

**Revendications**

**1.** 1. Dispositif pour analyser un modèle de déplacement enregistré au moyen d'un certain nombre de marqueurs ($M_i$) disposés sur les épaules et sur la tête d'un sujet à tester, comprenant une installation de traitement de données (3) reliée à un dispositif récepteur (2) pour l'enregistrement de la courbe locale ($m_i$) de chaque marqueur ($M_i$), qui comprend un niveau de traitement (31) pour le calcul d'un ensemble de données (DS) représentant la courbe locale ($m_i$) à partir de signaux du dispositif récepteur (2),
  **caractérisé en ce que**
l'installation de traitement de données (3) comprend un module d'analyse (33) avec un niveau de soustraction (33b) qui est conçu pour former à l'aide de l'ensemble de données (DS) la différence entre la valeur moyenne des deux courbes locales ($m_2$, $m_3$) représentant les déplacements de l'épaule et la courbe locale ($m_1$) représentant le déplacement de la tête et générer dans au moins l'une des trois coordonnées dans l'espace (x, y, z) la courbe du modèle de déplacement du cou qui en est déduit ($\Delta x$, $\Delta y$, $\Delta z$).

**2.** Dispositif selon la revendication 1,
  **caractérisé en ce que**
le dispositif récepteur comprend deux récepteurs (2) disposés à angle droit l'un par rapport à l'autre.

**3.** Dispositif selon la revendication 1 ou 2,
  **caractérisé en ce que**
le niveau de traitement (31) attribue la courbe locale ($m_i$) de chaque marqueur ($M_i$) comme une zone de données ($DF_i$) à l'ensemble de données (DS).

**4.** Dispositif selon l'une quelconque des revendications 1 à 3,
  **caractérisé en ce qu'**une mémoire temporaire d'ensemble de données (32) est disposée en aval du niveau de traitement (31).

**5.** Dispositif selon l'une quelconque des revendications 1 à 4,

**caractérisé en ce que**

l'installation de traitement de données (3) amène la courbe du modèle de déplacement du cou ($\Delta$x, $\Delta$y, $\Delta$z), calculé par la formation de la différence à un module de sortie (4) pour la représentation du modèle de déplacement.

Fig. 1

Fig. 2.1

Fig. 2.2

Fig. 2.3

Fig. 2.4

Fig. 3.2

Fig. 3.4

Fig. 3.1

Fig. 3.3

Fig. 4.1

Fig. 4.2

Fig. 4.3

Fig. 4.4

EP 1 171 034 B1

Fig. 5.1

Fig. 5.2

Fig. 5.3

Fig. 5.4

EP 1 171 034 B1

Fig. 6

Fig. 7

Fig. 8

EP 1 171 034 B1

Fig. 9

EP 1 171 034 B1

Fig. 10